# EUROPEAN PATENT SPECIFICATION

(11) **EP 2 129 337 B1**
(45) Date of publication and mention of the grant of the patent: **18.01.2017**
(21) Application number: 08725811.7
(22) Date of filing: 19.02.2008
(51) Int. Cl.: A61F 2/38

(54) **TISSUE SPARING IMPLANT**
GEWEBESCHONENDES IMPLANTAT
IMPLANT ÉPARGNANT DES TISSUS

(30) Priority: 16.02.2007 US 901846 P; 06.04.2007 US 922134 P; 01.05.2007 US 799474
(43) Date of publication of application: 09.12.2009
(73) Proprietor: Concept, Design And Development, Llc, Chagrin Falls, OH 44023 (US)
(72) Inventor: RIBOT, Michael, Baulkham Hills, NSW 2153 (AU); MCTIGHE, Timothy, Chagrin Falls, OH 44023 (US); HENRY, Jim, Edmond, OK 73013 (US); BANKS, Steve, Baulkham Hills, NSW 2153 (AU)
(74) Representative: Walker, Ross Thomson
(86) International application number: PCT/US2008/002217
(87) International publication number: WO 2008/100639

(56) References cited:
- EP-A1- 0 240 815
- WO-A1-97/33538
- DE-A1- 10 036 985
- FR-A1- 2 327 757
- US-A- 4 932 974
- US-A- 5 156 624
- US-A- 5 755 811
- US-A- 5 888 210
- US-A1- 2002 065 562
- US-A1- 2004 010 319

## Description

### BACKGROUND

### 1. The Field of the Invention.

The present disclosure relates generally to an orthopedic implant for use in a Primary Total Hip Arthroplasty, i.e., a total hip replacement. More specifically, the disclosure relates to a femoral component of a total hip implant, and more particularly, but not necessarily entirely, to a femoral neck sparing stem that may be placed or located in a medullary canal of the femur.

### 2. Description of Related Art.

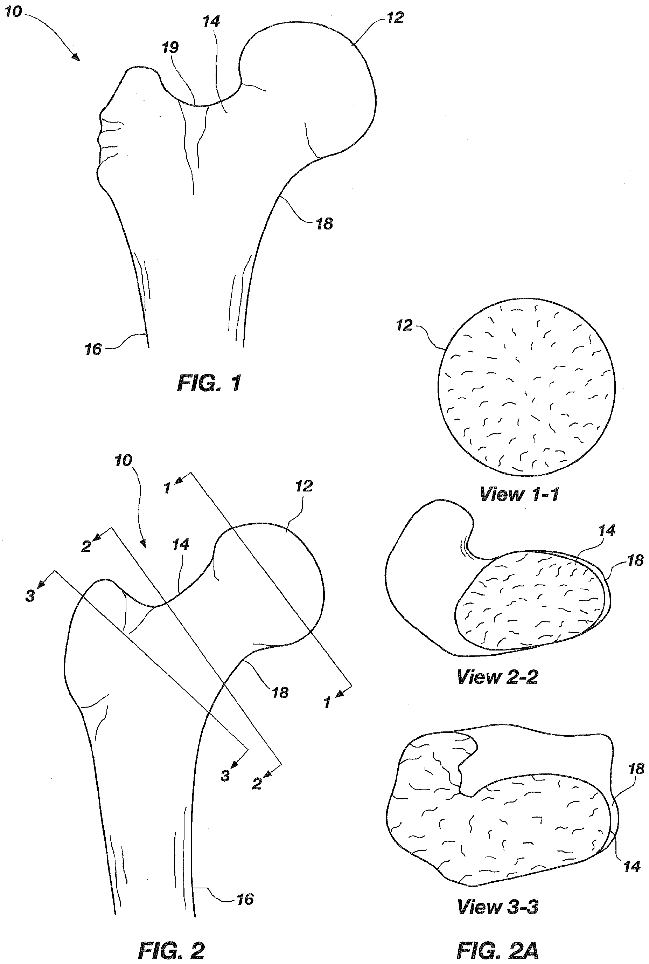

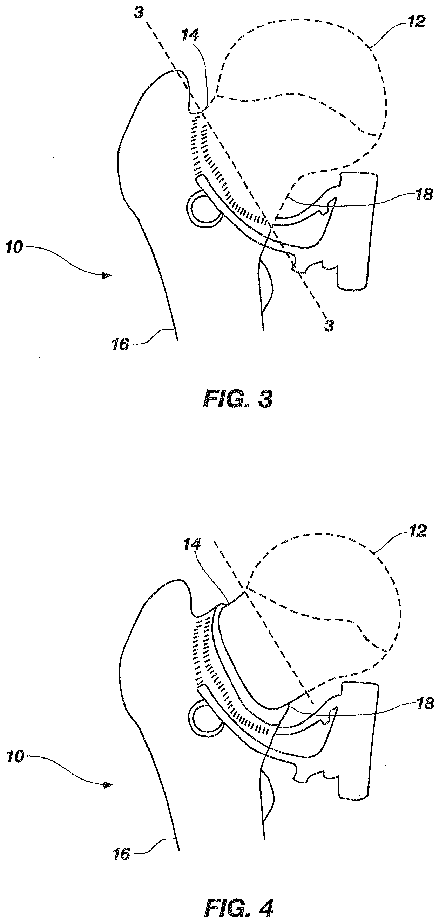

Hip implants are well known in the orthopedic industry. Referring now to FIGS. 1-4, in a Total Hip Arthroplasty ("THA"), a natural femur 10, which includes a femoral head 12, neck 14 and shaft 16, is surgically prepared to receive a femoral component of a hip implant. In preparing the femur 10 to receive the artificial femoral component, orthopedic surgeons often resect or remove the femoral head 12 from the natural femur 10. In removing the femoral head 12, there are generally two types of resections that may be performed, conventional and neck sparing. The difference in the two types of resections are illustrated best in FIGS. 2, 2A, 3 and 4, where a conventional resection is illustrated by the line 3-3 in FIG. 2 and in FIG. 3. Conversely, the neck sparing resection is illustrated in FIGS. 2 and 4 and the actual cut may occur just below line 1-1 in FIG. 2.

It will be appreciated that the type of hip implant used in a THA is largely dependent upon the type of resection of the femoral head 12 implemented. Neck sparing resections may be preferred if proper loading of the medial, proximal portion 18 of the femur 10, i.e., the medial calcar portion of the femur 10, occurs (see reference numeral 18 in FIGS. 2 and 2A), because more bone is spared thereby and is preserved for future use if a revision surgery is later required due to: infection, implant failure or otherwise. If a future revision surgery is necessary, then a conventional resection may be used at that time if the original resection was neck sparing. A neck sparing approach may thereby result in extending the mobility of a patient for an additional time period, which may be an additional 20 years or more.

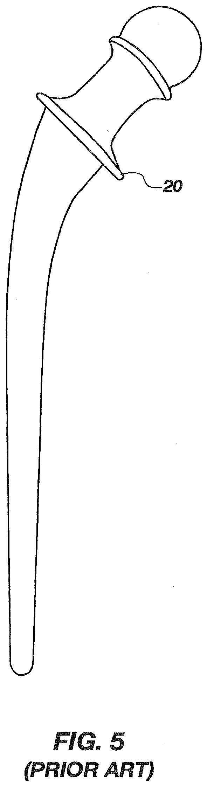

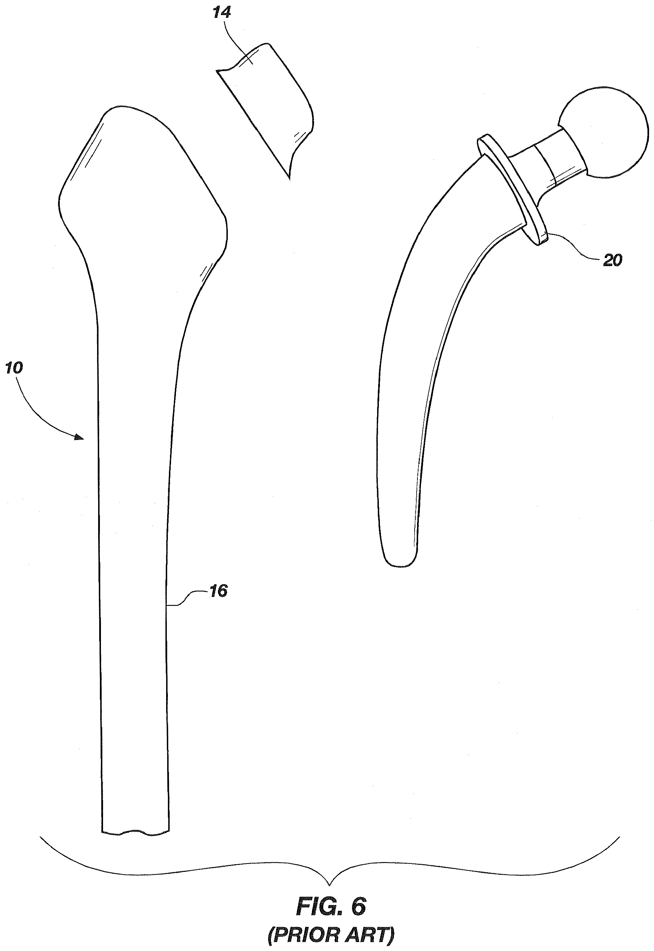

FIG. 5 illustrates a femoral component of a hip implant that may be used when a conventional resection or cut is made. FIG. 6 illustrates the amount of bone in the femoral neck 14 that may be spared using a neck sparing resection and a known femoral component of a hip implant that may be used when a neck sparing resection or cut is made.

It is to be understood that a natural bone is loaded from the outside in where the harder, more dense cortical bone is located. Conversely, an orthopedic implant changes the nature of the loading of the natural bone due to the hard, typically metallic, stem located within the femoral canal. Thus, an implant changes the natural loading of the bone from the outside in, to the inside out, as the load follows the stem and works outward therefrom. Further, if a bone is improperly loaded, then the bone will resorb, thereby providing aseptic loosening and failure of the implant. Thus, it is of utmost importance to load a bone properly to increase the efficacy of an implant.

It will be appreciated that the overall size and geometric shape of the femoral components illustrated in FIGS. 5 and 6 differ in large part due to the removal or maintenance of the femoral neck 14 portion of the bone because of the need, or lack thereof, to load the medial, proximal portion 18 of the femur 10.

DE 10036985 A1 discloses a femur component of an artificial hip joint.

Despite the advantages and longevity of THA implants, improvements are still being sought. Current implants on the market today are characterized by several disadvantages that may be addressed by the present disclosure. For example, neck sparing implants and devices on the market have traditionally had difficulty properly loading the bone and particularly the medial calcar portion of the femur. Thus, neck sparing devices have not realized their full potential for use in THA surgeries. The present disclosure minimizes, and in some aspects eliminates, the above-mentioned failures in neck sparing implants and devices, and other problems, by utilizing the methods and structural features described herein.

The features and advantages of the disclosure will be set forth in the description which follows, and in part will be apparent from the description, or may be learned by the practice of the disclosure without undue experimentation. The features and advantages of the disclosure may be realized and obtained by means of the instruments and combinations particularly pointed out in the appended claims.

### SUMMARY OF THE INVENTION

The present invention provides a tissue sparing implant according to claim 1. Any subject matter described herein that does not fall within the scope of claim 1 is provided for information purposes only.

### BRIEF DESCRIPTION OF THE DRAWINGS

The features and advantages of the disclosure will become apparent from a consideration of the subsequent detailed description presented in connection with the accompanying drawings in which:
FIG. 1 is a side, cross-sectional view of a natural femoral bone illustrating areas of dense cortical bone;
FIG. 2 is a side view of a natural femoral bone; FIG. 2 A illustrate three cross-sections of the femur taken along the lines 1-1, 2-2 and 3-3 of FIG. 2;
FIG. 3 is a side view of a natural femur illustrating a conventional neck resection made in a total hip arthroplasty surgery, with the neck and head of the femur in phantom;
FIG. 4 is a side view of a natural femur illustrating a neck sparing resection made in a total hip arthroplasty surgery, with only the head in phantom;
FIG. 5 illustrates a known conventional stem used in conjunction with a conventional femoral neck resection illustrated in FIG 3;
FIG. 6 illustrates a known neck sparing device and further illustrates how much of the femoral neck can be spared using such a device in comparison to a conventional stem of FIG. 5;
FIG. 7 is a side view of the implant of the present disclosure made in accordance with the principles of the present disclosure;
FIG. 8 is a side view of a natural femur and illustrates the difference and amount of bone spared between a neck sparing resection and a conventional resection;
FIG. 9 is a top, perspective view of one embodiment of a femoral stem component having a lateral fin made in accordance with the principles of the present disclosure;
FIG. 10 is a top view of the embodiment of the femoral stem component of FIG. 9 made in accordance with the principles of the present disclosure;
FIG. 11 is a side view of the embodiment of the femoral stem component of FIG. 9 made in accordance with the principles of the present disclosure;
FIG. 12 is a front view of the embodiment of the femoral stem component of FIG. 9 made in accordance with the principles of the present disclosure;
FIG. 13 is a bottom, perspective view of the embodiment of the femoral stem component of FIG. 9 made in accordance with the principles of the present disclosure;
FIG. 14 is a top, backside perspective view of another embodiment of a femoral stem component having a T-back or wing back made in accordance with the principles of the present disclosure;
FIG. 15 is a side view of the embodiment of the femoral stem component of FIG. 14 made in accordance with the principles of the present disclosure;
FIG. 16 is a front side, perspective view of the embodiment of the femoral stem component of FIG. 14 made in accordance with the principles of the present disclosure;
FIG. 17 is a bottom, perspective view of the embodiment of the femoral stem component of FIG. 14 made in accordance with the principles of the present disclosure;
FIG. 18 is a front view of the embodiment of the femoral stem component of FIG. 14 made in accordance with the principles of the present disclosure;
FIG. 18A is a front view of the embodiment illustrated in FIG. 18;
FIG. 18B is a side view of the embodiment illustrated in FIG. 18;
FIG. 18C is a top view of the embodiment illustrated in FIG. 18;
FIG. 18D is a sectional view taken along the line E-E of FIG. 18B;
FIG. 18E is a sectional view taken along the line H-H of FIG. 18A;
FIG. 18F is another top view taken along the line F-F of FIG. 18B;
FIG. 18G is a sectional view taken along the line G-G of FIG. 18F;
FIG. 18H is a sectional view taken along the line K-K of FIG. 18E;
FIG. 18I is a sectional view taken along the line J-J of FIG. 18E;
FIG. 19 is another top, backside perspective view of the embodiment of the femoral stem component of FIG. 14 made in accordance with the principles of the present disclosure;
FIG. 20 is a side view of another embodiment of a femoral stem component made in accordance with the principles of the present disclosure;
FIG. 21 is a front view of the embodiment of the femoral stem component of FIG. 20 made in accordance with the principles of the present disclosure;
FIG. 22 is a side view of another embodiment of a femoral stem component made in accordance with the principles of the present disclosure;
FIG. 23 is a front view of the embodiment of the femoral stem component of FIG. 22, illustrating the T-back or wing back, and made in accordance with the principles of the present disclosure;
FIG. 24 is a side view of another embodiment of a femoral stem component made in accordance with the principles of the present disclosure illustrating a sagittal slot and a tapered stem concept;
FIG. 25 is a front view of the embodiment of the femoral stem component of FIG. 24 made in accordance with the principles of the present disclosure;
FIG. 26 is a side view of another embodiment of a femoral stem component made in accordance with the principles of the present disclosure illustrating the lateral fin concept;
FIG. 27 is a front view of the embodiment of the femoral stem component of FIG. 26 made in accordance with the principles of the present disclosure;
FIG. 28 is a side view of another embodiment of a femoral stem component made in accordance with the principles of the present disclosure;
FIG. 29 is a back view of the embodiment of the femoral stem component of FIG. 28 made in accordance with the principles of the present disclosure;
FIG. 30 is a perspective view of the embodiment of the femoral stem component of FIG. 28 made in accordance with the principles of the present disclosure;
FIG. 31 is a bottom perspective view of the embodiment of the femoral stem component of FIG. 28 made in accordance with the principles of the present disclosure;
FIG. 32 is a side perspective view of the embodiment of a modular head component and a modular neck component of the femoral stem component of FIG. 28 made in accordance with the principles of the present disclosure;
FIG. 33 is a side view of the embodiment of the femoral stem component of FIG. 28, illustrating one embodiment of a modular neck component and made in accordance with the principles of the present disclosure;
FIG. 34 is a perspective side view of a monoblock head and neck component of the femoral stem component of FIG. 28 made in accordance with the principles of the present disclosure;
FIG. 35 is a top, front view of a modular neck component of FIG. 32 made in accordance with the principles of the present disclosure; and
FIG. 36 is a bottom, side perspective view of a modular neck embodiment made in accordance with the principles of the present disclosure.

### DETAILED DESCRIPTION

For the purposes of promoting an understanding of the principles in accordance with the disclosure, reference will now be made to the embodiments illustrated in the drawings and specific language will be used to describe the same. It will nevertheless be understood that no limitation of the scope of the disclosure is thereby intended. Any alterations and further modifications of the inventive features illustrated herein, and any additional applications of the principles of the disclosure as illustrated herein, which would normally occur to one skilled in the relevant art and having possession of this disclosure, are to be considered within the scope of the disclosure claimed.

It is to be understood that this disclosure is not limited to the particular configurations, process steps, and materials disclosed herein as such configurations, process steps, and materials may vary somewhat. It is also to be understood that the terminology employed herein is used for the purpose of describing particular embodiments only and is not intended to be limiting since the scope of the present disclosure will be limited only by the appended claims and equivalents thereof.

It must be noted that, as used in this specification and the appended claims, the singular forms "a," "an," and "the" include plural referents unless the context clearly dictates otherwise.

In describing and claiming the present disclosure, the following terminology will be used in accordance with the definitions set out below.

As used herein, the terms "comprising," "including," "containing," "characterized by," and grammatical equivalents thereof are inclusive or open-ended terms that do not exclude additional, unrecited elements or method steps.

As used herein, the phrase "consisting of" and grammatical equivalents thereof exclude any element, step, or ingredient not specified in the claim.

As used herein, the phrase "consisting essentially of" and grammatical equivalents thereof limit the scope of a claim to the specified materials or steps and those that do not materially affect the basic and novel characteristic or characteristics of the claimed disclosure.

As used herein, the term "proximal" shall refer broadly to the concept of a portion nearest to the center of a reference point, such as a patient's body, or a "point of origin" as that phrase is known in the medical field. For example, a natural femoral bone includes a proximal end having a femoral head that forms part of a hip joint proximally and a distal end having femoral condyles that form part of the knee joint distally. Thus, the proximal femur is so named because it is the proximal-most portion of the femur and is nearest to the center of the patient's body. As another example, a patient's knee is proximal with respect to the patient's toes.

On the other hand, as used herein, the term "distal" shall generally refer to the opposite of proximal, and thus to the concept of a portion farthest from the center of a patient's body, depending upon the context. Thus, the distal femur, for example, is so named because it is the distal-most portion of the femur and is farthest from the center of the patient's body. As another example, a patient's fingers are distal with respect to the patient's shoulder, if the shoulder is the reference point.

As used herein, the phrase "in an at least partially proximal-to-distal direction" shall refer generally to a two-dimensional concept of direction in which the "proximal-to-distal" direction defines one direction or dimension. An item that extends in a non-parallel direction with respect to the "proximal-to-distal" direction, that is, at a non-straight angle thereto, thereby involves two components of direction, one of which is in the "proximal-to-distal" direction and the other having some other component of direction, for example a direction orthogonal to the "proximal-to-distal" direction. As a specific example, a patient's natural femur extends in a substantially proximal-to-distal direction.

It will be appreciated that FIGS. 1-6 generally illustrate features of the femoral bone and the various resections that may be used in a total hip arthroplasty surgery. For example, FIGS. 1-2A illustrate the bone structure of the proximal femur, and particularly the head 12, neck 14 and medial calcar 18 sections of the femur 10. On the other hand, FIGS. 3-4 illustrate a conventional resection versus a neck sparing resection of the femoral head and FIGS. 5-6 illustrate a known conventional prosthetic stem versus a known neck sparing prosthetic stem for purposes of demonstrating the amount of bone that may be preserved or spared using a neck sparing resection.

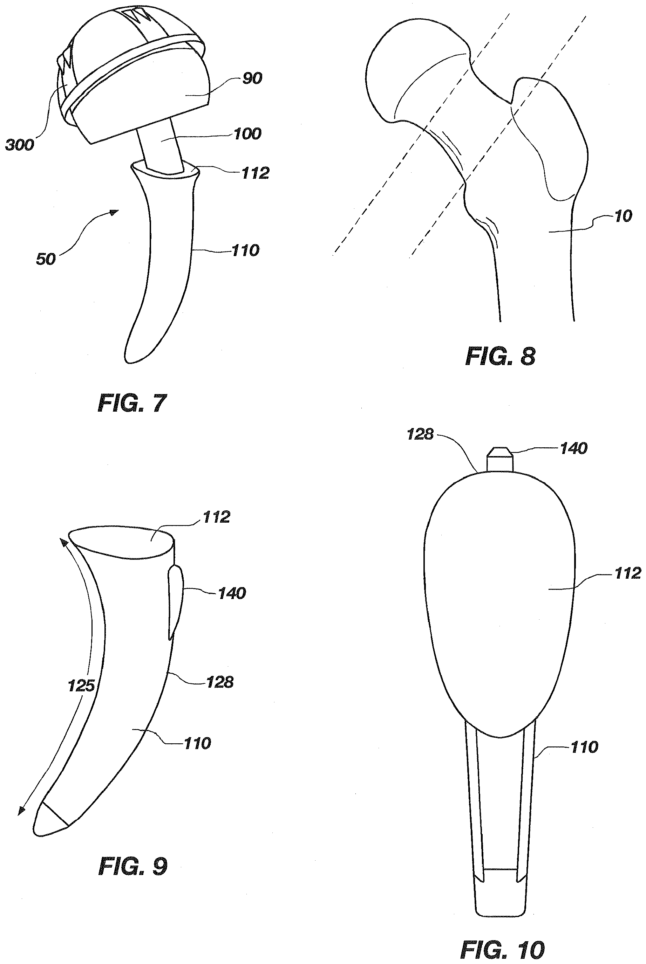

Referring generally to FIGS. 7-27, the present disclosure may be directed to a hip implant 50 and specifically to a femoral component 110 that may be designed to properly load the medial, proximal portion 18 of the femur 10. By utilizing the unique geometric design features discussed and disclosed herein, loads placed on the femoral side of the hip joint may be properly distributed to the medial, proximal portion 18 of the femur 10, thereby maintaining bone strength at that location.

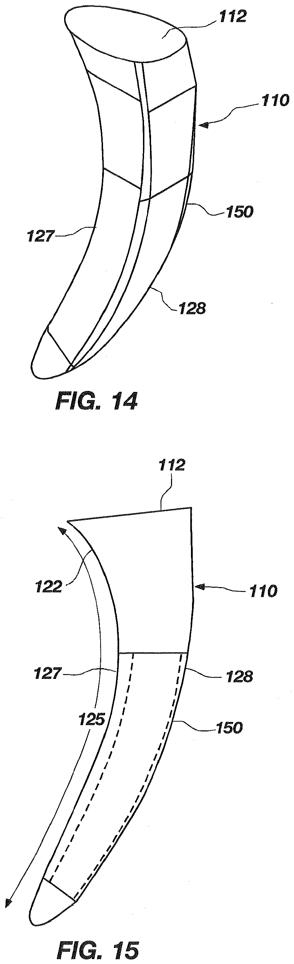

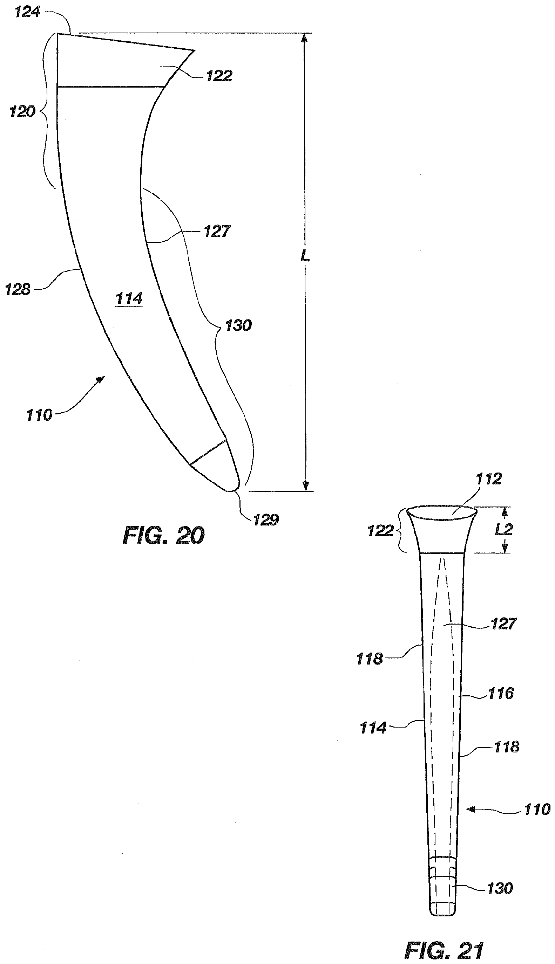

There are several embodiments of the femoral component 110 of the present disclosure, all of which share the major features of the disclosure. A first embodiment (known as the lateral fin stem) of the femoral component 110 is illustrated in FIGS. 9-13 and 26-27. Whereas, a second embodiment (known as the T-back or wing back stem) of the femoral component 110 is illustrated in FIGS. 14-19 and 22-23. Other embodiments of the femoral component 110 are illustrated in FIGS. 20-21 and 24-25.

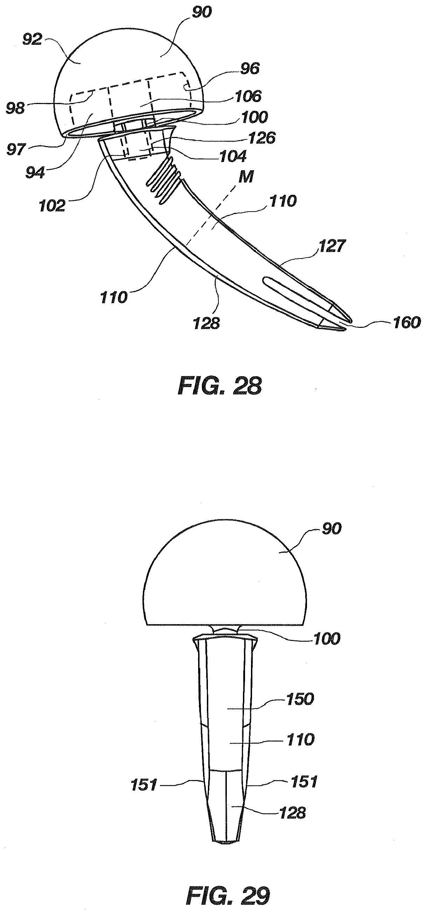

Referring specifically now to the features in common to all embodiments of the present disclosure and to FIGS. 9-19, the femoral or stem component 110 may include a proximal body portion or component 120 and a distal stem portion or component 130. The proximal body portion 120 may include a terminal flare portion 122, extending from a proximal end 124 of the stem component 110 for a distance that is sufficient to enable the terminal flare portion 122 to contact a medial calcar portion of the femur internally, thereby distributing load from said stem component 110 to the bone. The terminal flare 122 may terminate in a free, at least partially circumferential, edge as illustrated for example in FIG. 28-31. In other words, the terminal flare portion 122 may be designed to be located internally with respect to the femoral bone and may be considered a collarless stem. The phrase "terminal flare" as used herein shall therefore refer to the foregoing features, and also in reference to a flare terminating in a free and at least partially circumferential edge or edge section and in either case unencumbered and otherwise not directly attached to a device or item that extends radially further than said at least partially circumferential edge or edge section. As such, an implant having a collarless proximal portion that has a terminal flare, is configured and arranged to limit its contact with the femur to internal portions of the femur, and without structure that would operate to contact external portions of the femur, and further without a collar or other structure that would operate to contact portions of the femur that reside above and radially outward from the terminal portion of said terminal flare.

In contrast, an external collar 20 or a stem containing a collar 20, as illustrated in FIG. 5, is not a terminal flare due to the presence of a collar and may be located externally with respect to the bone and may therefore operate to place the load externally with respect to the bone. Another example of an external collar 20 is illustrated in FIG. 6 and is not the same as a terminal flare portion 122, which may be designed to be located internally (not externally) with respect to the bone. External collars 20 may be designed to place an axial, downward load on a resected surface of the bone, which may not load the medial calcar portion of the femur. Whereas, the terminal flare portion 122 may function to load the medial, proximal portion 18 of the femur 10 internally, such that a much larger portion of the medial, proximal portion 18 of the femur may be loaded, thereby reducing the risk that portion of the bone will resorb.

Referring to FIG. 7, the tissue sparing implant 50 of the present disclosure may include a head component 90, a neck component 100 and a stem component 110. The various components of the implant 50 may be manufactured from various materials, including implant grade alloys, such as titanium and its alloys and chrome cobalt and alloys containing chrome cobalt. It will be appreciated that the implant 50 and its various components may be manufactured from any bio-compatible material without limitation and without departing from the scope of the present disclosure.

The head component 90 may generally be a convex surface for articulating with a concave surface located opposite the head component 90 formed, for example, as part of an acetabular component 300. In other words, the head component 90 may be configured and dimensioned to articulate with an acetabular component 300 of an artificial hip implant 50.

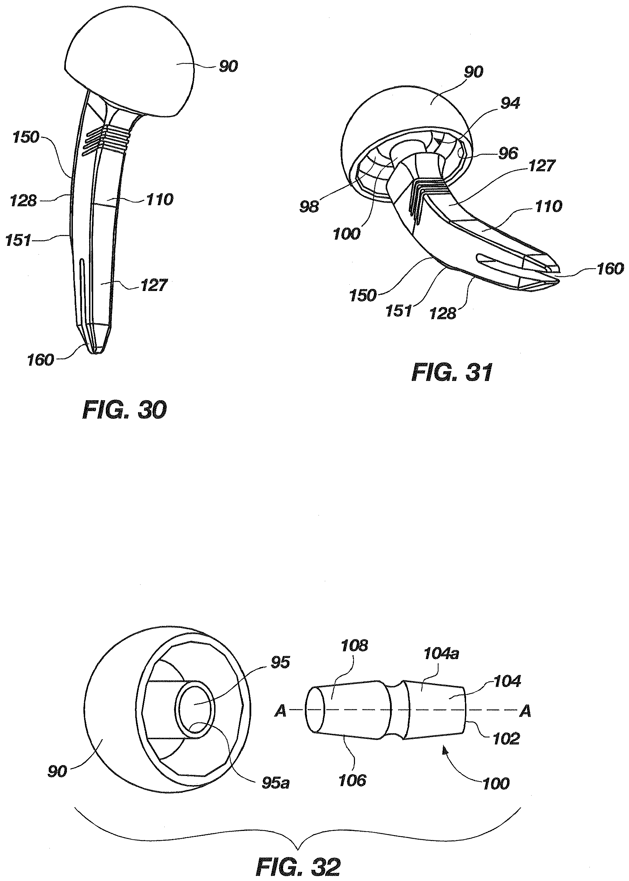

Referring now to FIGS. 28-36, the head component 90 may be modular or it may be formed as a unitary piece with respect to the neck component 100. When modular, the head component 90 may include a recess 95 (illustrated best in FIG. 32). The recess 95 may be defined by a tapered sidewall 95a for use in attaching the head component 90 to the neck component 100 in a tapered fitting. It will be appreciated that the recess 95 may be present in the modular embodiment (with respect to the neck component 100), but may not be present in the unitary embodiment, since in the unitary embodiment no modular connection is necessary.

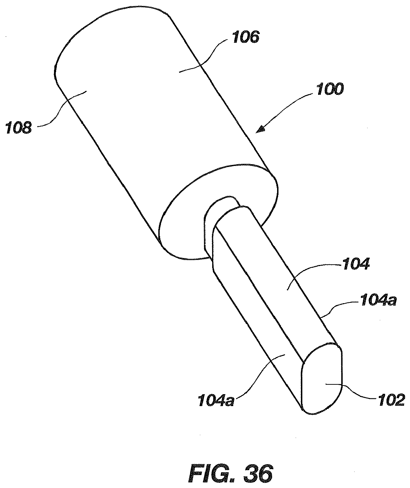

In the modular embodiment, the neck component 100 may include a tapered end portion 108 (illustrated best in FIGS. 32 and 36) that may be insertable into the recess 95 of the head component 90 (illustrated best in FIG. 32), thereby securing the head component 90 to the modular neck component 100 via a morse tapered friction fit. For example, the tapered end portion 108 of the neck component 100 and the recess 95 of the head component 90 may each be substantially cylindrical in shape and may be tapered for matingly engaging each other. The tapered fitting between the end portion 108 and the matching tapered wall 95a of the recess 95, may include a 12:14 taper ratio. However, other taper ratios may be utilized without departing from the scope of the present disclosure. It will be appreciated that such a tapered connection is only one example of a modular connection. Modular connections are well known in the orthopedic field and any type of modular connection may be used to attach the head component 90 to the neck component 100 without departing from the spirit or scope of the present disclosure.

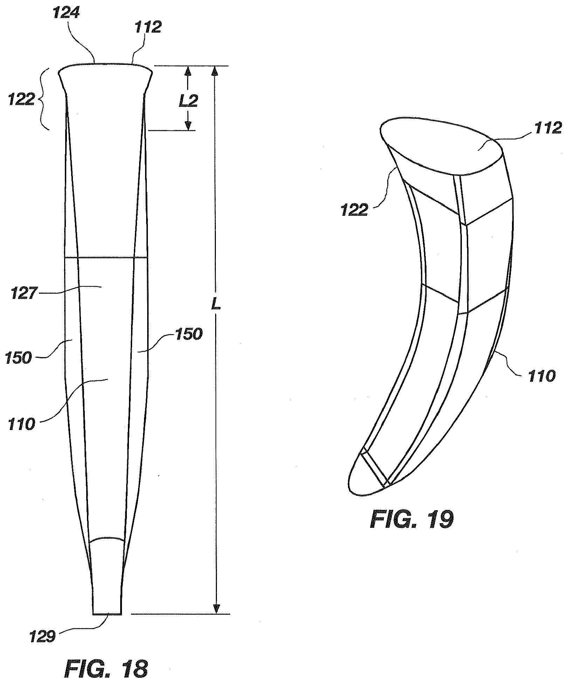

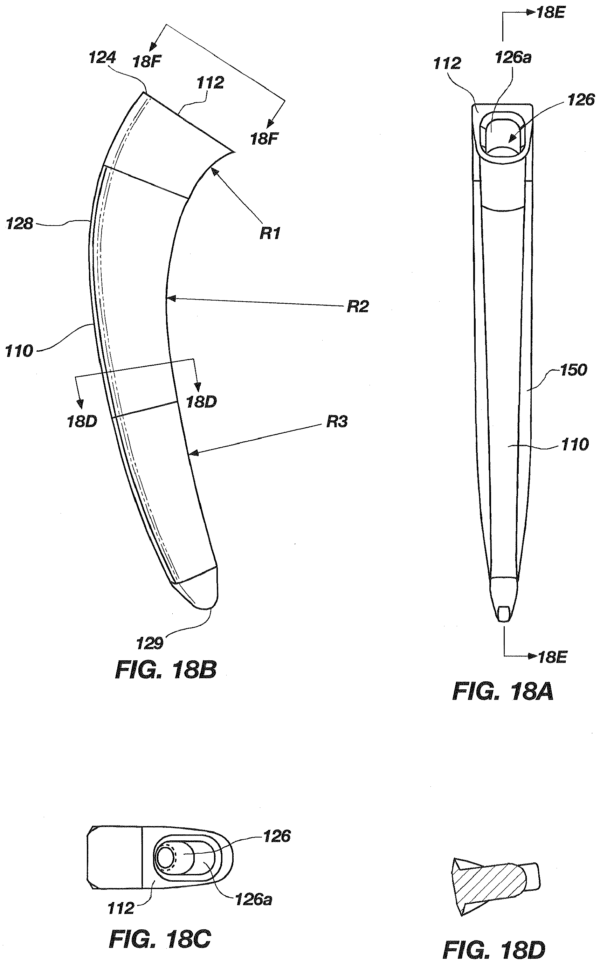

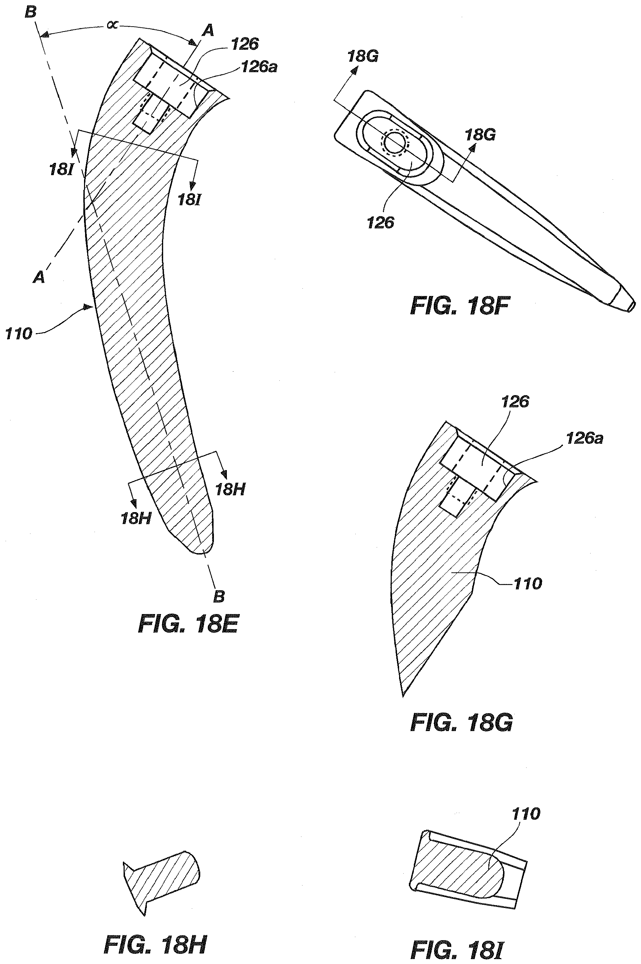

In another embodiment (illustrated best in FIGS. 28-31), the head component 90 may be formed as a unitary piece with the neck component 100, instead of the head component 90 being a modular piece with respect to the neck component 100. In other words, the head component 90 may be formed or manufactured as a single, unitary piece with the neck component 100, i.e., the two elements may be formed in a monoblock design, such that the only modular connection is with respect to a distal end 102 of the neck component 100 that may be modularly attached to a recess 126 formed in a top surface of the proximal body portion 120 of the stem component 110. The recess 126 is best illustrated in FIGS. 18A, 18C, 18F, 18G and 18H.

It will be appreciated that the neck component 100, whether in a monoblock or a modular embodiment with respect to the head component 90, may be variable in length and variable in angle to modify or correct the version and lateral offset of the head component 90. The ability to correct version and lateral offset of the head component 90 allows for the reproduction of a patient's natural anatomical features. More specifically, the head component 90 and neck component 100, whether monoblock or modular, may be manufactured in a neutral manner, which may be used in either a right or left hip joint, or may be manufactured as a left implant used in a patient's left hip joint, or as a right implant used in a patient's right hip joint. Thus, the neck component 100, whether monoblock or modular with respect to the head component 90, may be available in different lengths and different models, which may affect a patient's leg length, varus and valgus orientation, and anteversion or retroversion, or a combination of all three.

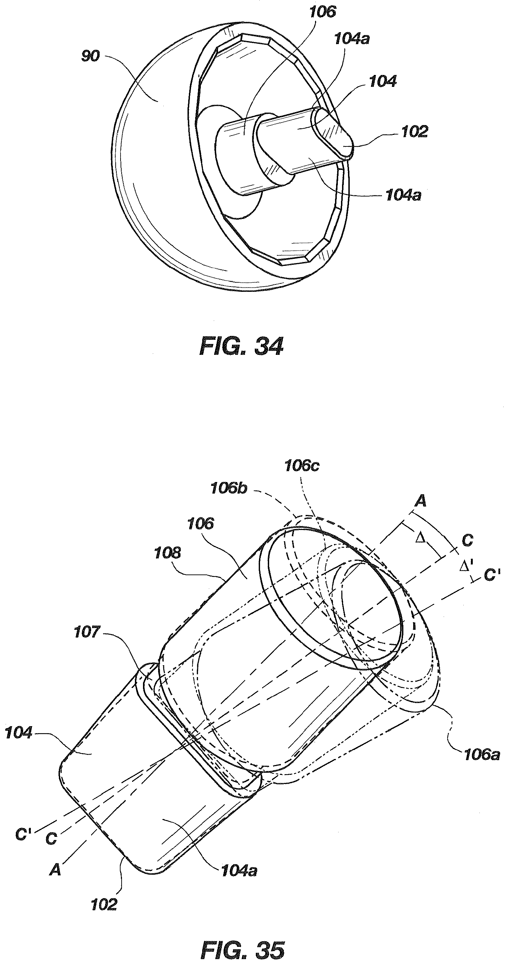

Further, the neck component may include a shaft 106, whether monoblock or modular with respect to the head component 90, which may be formed in a substantially upright or axial manner with respect to a central neck axis A-A in a neutral neck component 100 (illustrated best in FIG. 35). Alternatively, the shaft 106 may bend at a junction 107 where the shaft 106 meets a modular attachment 104 (see FIG. 35) to form an angle Δ (illustrated in phantom lines in FIG. 35). The angle Δ may be formed by a central axis (illustrated as lines C-C and C'-C' in FIG. 35) of the bent shaft 106 and a central neck axis A-A, which may extend centrally through the modular attachment 104, as illustrated in FIG. 35. The angle Δ or Δ' may be formed within a range of about four degrees to about twenty four degrees (or any angle within that range), and more specifically between a range of about four degrees to about eight degrees. It will be appreciated that the various angles may result in a different center of rotation of the joint.

No matter which embodiment of the head component 90 is utilized or chosen, i.e., whether modular or monoblock with respect to the neck component 100, the head component 90 may be sized between about 22 millimeters to about 60 millimeters in diameter and may include all sizes between 22 millimeters and 60 millimeters. For example, as illustrated in FIGS. 28-31, the head component 90 may be oversized and may fall within a range of about 28 millimeters to about 60 millimeters in diameter, and more specifically between a range of about 32 millimeters and about 60 millimeters in diameter. The larger head diameter may function to increase the stability of the entire femoral component 110 within the acetabular component 300 (illustrated best in FIG. 7).

It will be appreciated that the neck component 100, whether part of a monoblock or modular design with respect to the head component 90, may be anteverted. The anteversion and offset may be adjusted by a surgeon during a particular surgery to create the best possible fit for the patient due to the features of the present disclosure. It should be noted that the size and shape of the modular pieces of the implant, i.e., the head component 90 and the neck component 100, may affect offset. Similarly, the size and shape of the monoblock embodiment of the head component 90 and neck component 100 may also affect offset. Thus, the anteversion as well as the head size and shape may increase or vary the offset, which is the distance between a longitudinal stem axis and a center of rotation within the hip joint. For example, using a larger head 125 increases the distance between the center of rotation and the longitudinal stem axis and thus may increase the offset.

The head component 90 illustrated in FIGS. 28-31 may include a convexly shaped outer surface portion 92 and a recessed area 94 that may be formed substantially opposite the convexly shaped outer surface portion 92. The recessed area 94 may be defined by an inner sidewall 96 (illustrated best in FIGS. 28 and 31) that may extend from a rim or base 97 of the head component 90 for a distance and may terminate in an upper, inner surface 98.

Referring now to the neck component 100 of the monoblock head/neck embodiment, the neck 100 may comprise a modular attachment 104 at its distal end 102. It will be appreciated that the modular attachment 104 may be any modular attachment known, or that may become known, in the art without departing from the scope of the present disclosure. One exemplary embodiment of the modular attachment 104 includes an oblong cross-sectional shape, which may include a substantially rectangular cross-sectional shape as illustrated in FIG. 34.

Another exemplary embodiment of the neck component 100, whether the monoblock or modular embodiment with respect to the head component 90, may include a reverse trunnion shape as illustrated in FIGS. 34-36. In either embodiment, the neck component 100 may include the shaft 106, which may be essentially cylindrical or otherwise shaped. The neck component 100 may also include the modular attachment 104, which may be received in the recess 126 of the proximal body portion 120. The modular attachment 104 may include two substantially flat side portions 104a (illustrated best in FIGS. 34-36) that may be shaped to match the shape of the recess 126 formed in the proximal body portion 120 of the stem component 110. In cross-section, the attachment piece 104 may be shaped in an oblong manner, and more specifically may be shaped in a rectangular manner. The modular attachment 104 may also taper and may matingly engage a sidewall 126a of the recess 126, which may also taper. It will be appreciated that other cross-sectional shapes may also be utilized as the shape of the attachment piece 104 without departing from the spirit or scope of the present disclosure.

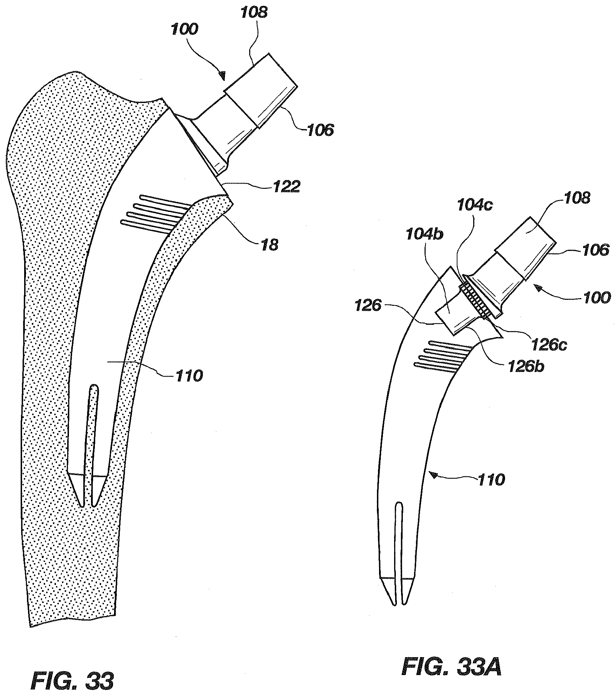

Referring to FIGS. 33 and 33A, in an alternative embodiment, the modular attachment 104 of the neck component 100 may include a double taper 104b (illustrated best in FIG. 33A) to attach the neck component 100 to the recess 126. It will be appreciated that the recess 126 may be similarly shaped and may comprise a double tapered sidewall 126b, such that there may be a mating engagement between the double taper 104b of the neck component 100 and the double tapered sidewall 126b of the recess 126. The neck component 100 may also include an indexing feature, which may be in the form of a series of teeth 104c that may mate with corresponding teeth 126c in the sidewall 126b of the recess 126 to allow the head component 90 and the neck component 100 to be oriented in one of a plurality of orientations. It will be appreciated that the neck component 100 may be anteverted, such that when oriented in one of, for example, twelve different positions within the recess 126, the angle of anteversion may be modified and adjusted by a surgeon during a surgical procedure.

Thus, the neck component 100 may include an attachment 104 that may be configured and dimensioned for insertion into the recess 126 of the stem component 110 to thereby secure the neck component 100 to the stem component 110. The neck component 100 may be secured and attached to the stem component 110 via a means for securing the neck component 100 to the stem component 110. It will be appreciated that the means for securing the neck component 100 to the stem component 110 may be any type of modular connection known in the art, or which may become known in the art in the future, without departing from the spirit or scope of the present disclosure. Thus, the means for securing the neck component 100 to the stem component 110 may include a tapered connection, a key and hole connection, a bayonet connection, or other modular connection without departing from the spirit or scope of the present disclosure.

Referring to FIGS. 18E and 35, the modular neck component 100 may include a neck axis A-A that may extend centrally through the neck component 100 as illustrated best in FIG. 35. The neck axis A-A may, for example, be an imaginary line bisecting sequential geometric centroids of successive cross-sections of the neck component 100. However, the neck axis A-A may also refer to a line bisecting sequential geometric centroid sections of the neck component 100, wherein the phrase "centroid section" refers to a portion of a cross-section covering thirty-three percent of said cross-section and also containing said geometric centroid of said cross-section. It will be appreciated that the neck component 100 may be a modular neck component (FIG. 32) or the neck component 100 may be integrally formed with the head component 90 in a monoblock head/neck embodiment (FIG. 34) or may even be formed integrally with the stem component 110 in a monoblock stem embodiment. In a modular neck embodiment, the stem component 110 may be attachable to the neck component 100 and the stem component 110 may include a distal stem axis B-B that may extend longitudinally and centrally through a distal most end of the stem component 110 as illustrated in FIG. 18E. The stem axis B-B may, for example, be an imaginary line bisecting sequential geometric centroids of successive cross-sections of the stem component 110. However, the stem axis B-B may also refer to a line bisecting sequential geometric centroid sections of the stem component 110, wherein the phrase "centroid section" refers to a portion of a cross-section covering thirty-three percent of said cross-section and also containing said geometric centroid of said cross-section.

It will be appreciated that an angle α may be formed by an intersection of the neck axis A-A when attached to said stem component 110 (whether in a modular neck embodiment or a monoblock embodiment) and the distal stem axis B-B. The angle α may be within a range of about forty-five degrees and about sixty degrees (or any angle within that range) and the angle α may be configured to model the natural medial curvature of a femoral neck of a natural femoral bone 10. More specifically, the angle α may be within a range of about fifty degrees to about fifty-five degrees.

It will be appreciated that the angle α may be directly proportional to a medial curvature of the stem component 110, such that an increase in the curvature of the stem component 110 may result in a larger angle α. The medial curvature of the stem component 110 may be substantial with respect to the proximal most one-third of the stem component 110 due to the neck sparing resection of the proximal femur 10 and the need of the stem component 110 to model the natural medial curvature of the medial, proximal portion 18 of the femur 10.

Because the natural femur 10 includes a significant medial curvature at the proximal medial calcar region, a neck sparing implant 50 may need to model the curvature of the natural femur 10 at that location when the natural neck 14 of the femur 10 is spared. It will be appreciated that failure of the implant 50 may occur if the curvature of the natural femur 10 on the medial side is not modeled, followed, matched or mimicked. At least one of the reasons for failure of known neck sparing devices is due to the lack of medial curvature and lack of proper loading of the proximal, medial calcar region of the femur 10. In other words, without such a substantial medial curvature of the femoral component 110, which may be similar or substantially similar to the natural medial curvature of the medial calcar region of the natural femur 10, the femoral component 110 may not properly load the medial calcar of the femur 10 resulting in bone resorption and ultimately implant failure.

Referring now to the various stem components 110 of the present disclosure and the respective embodiments illustrated in FIGS. 7-31, generally the stem component 110 may include a top surface 112 that may be formed in a plane that may be substantially perpendicular to, or a plane that is substantially transverse to, the neck axis A-A when the neck 100 is attached to the stem component 110. It will be appreciated that the top surface 112 may not be formed completely perpendicular to the neck axis A-A as described above, and instead may be formed in a crosswise manner with respect to the neck axis A-A. The top surface 112 may be formed at a proximal end portion 124 of the stem component 110.

The neck component 100 may be modular with respect to the stem component 110, and, if so, the top surface 112 of the stem component 110 may include the recess 126, discussed previously, that may be configured and dimensioned to receive the neck component 100 therein, without regard to whether the head component 90 is modular with respect to the neck component 100.

Referring specifically to FIGS. 20-21, the stem component 110 may comprise an anterior side portion 114, a posterior side portion 116, a medial side portion 127, a lateral side portion 128, the proximal body portion 120 and a distal stem portion 130. The stem component 110 may include a shortened stem length, represented by the distance "L," that may be less than about 150 millimeters. It will be appreciated that the length "L" of the stem component 110 may be measured from a proximal most end 124 of the stem component 110 to a distal most end 129 of the stem component 110, as demonstrated in FIG. 20. More specifically, the length "L" of the stem component 110 may be within a range of about 100 millimeters to about 120 millimeters.

In addition, it will be appreciated that the femoral component 110 may be designed to include the distal stem portion 130 with a length that is substantially shorter than a conventional stem (illustrated in FIG. 5). In other words, the length of the present disclosure's distal stem portion 130 may be shorter than the conventional stem for use in conjunction with a neck sparing resection.

It will be appreciated that the anterior side portion 114 and the posterior side portion 116 may each comprise a flat surface 118, which may aid in resisting torsional forces in the hip joint. The flat surface 118 may be defined by a plane that may lie along the anterior or posterior side of the stem component 110 when it is implanted into a patient's body. Thus, flat surface 118 may be substantially planar or level. It will be appreciated that the substantially planar or flat surface 118 may extend substantially along the entire length "L" of the stem component 110 (as illustrated in FIG. 21), or alternatively the flat surface 118 may extend along a majority length "L" of the stem component 110. The flat surface 118 of the anterior side portion 114 and the posterior side portion 116 may function to provide torsional stability to the stem component 110 due to the blunt shape of the anterior and poster side portions 114 and 116. It should be noted that the bluntness of the anterior and posterior side portions 114 and 116 may only be with respect to the flat surface 118, and it should be noted that the anterior and posterior side portions 114 and 116 may contain rounded corners and edges to avoid

However, it will be appreciated that such a flat surface 118 may not be present on the stem component 110 and may not be necessary. Instead, the anterior side portion 114 and the posterior side portion 116 may each comprise a curved exterior shape or a convex exterior shape without departing from the spirit or scope of the present disclosure. If the flat surface 118 is not present, then it will be appreciated that other features may be added to the stem component 110 to increase torsional stability, since torsional forces are very common in hip implants and particularly in femoral components 110.

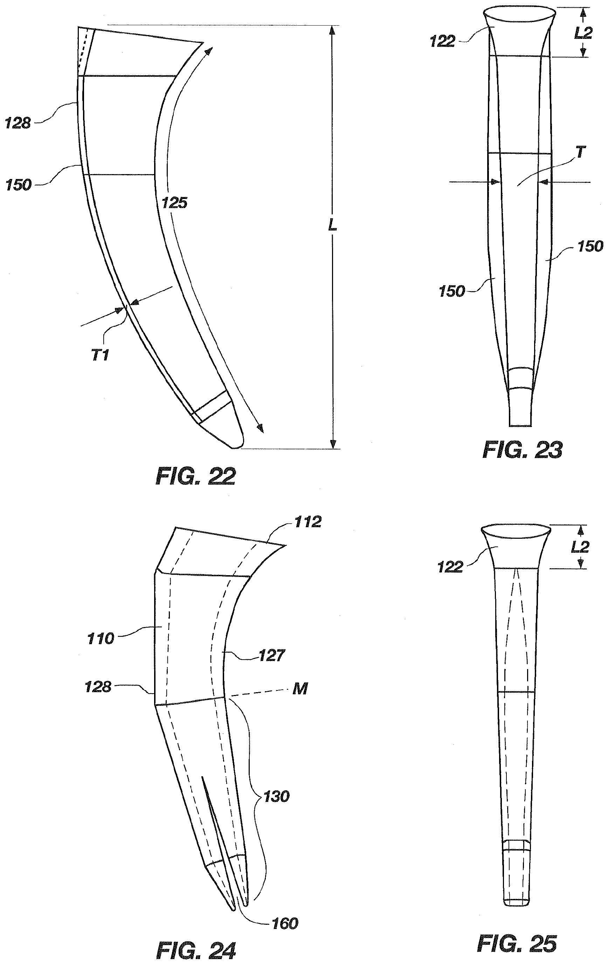

The stem component 110 may further comprise a curve (represented by the arc 125) on the medial side 127 of the stem component 110. The curve may extend along a majority length "L" of the stem component 110 on the medial side 127 as illustrated in FIGS. 18B and 22. The medial curve may include a plurality of different radii of curvature, and may include at least three different radii of curvature (illustrated best in FIG. 18B). The individual radii of curvature may each increase along the medial curve from the proximal end 124 of the stem component 110 to the distal end 129 the stem component 110. It will be appreciated that the medial curve may be configured and dimensioned to model the natural medial curvature of the femoral neck of the natural femur 10.

In other words, the various radii of curvature of the medial curve, represented in FIG. 18B by the reference numerals R1, R2 and R3, may increase such that R1 may have the smallest radius of curvature; R2 may have a radius of curvature that may be larger than R1; and R3 may have a radius of curvature that is larger than both R1 and R2. Thus, R3 may represent the largest radius of curvature along the medial curve. It will be appreciated that R1 may be located nearest the proximal most end 124 of the stem component 110 and may be followed by R2, which may be located nearest the midline of the medial side 127 of the stem component 110, and R2 may be followed by R3, which may be located nearest the distal end 129 of the stem component 110.

By way of specific example, as illustrated in FIG. 18B, the radius represented by R1 may be within a range of about .5 to about 1.0 inch, and may be about .750 inch. The radius represented by R2 may be within a range of about 2.3 to about 3.2 inches, and may be about 2.8 inches. The radius represented by R3 may be within a range of about 13.0 to about 14.5 inches, and may be about 13.780 inches.

Referring briefly now to FIG. 15, the medial side 127 may be described as having a substantial curvature (represented by the arc 125) along the entirety of its inner side. However, the substantial curvature 125 may be most pronounced in roughly the proximal most one-third (1/3), or along the proximal body portion 120, of the femoral component 110 on a medial side 127 of the stem component. Further, the substantial curvature represented by the arc 125 along the proximal body portion 120 may be designed to imitate or match the natural curvature of the proximal, medial portion 18 of the femur 10. Thus, the substantial curvature 125 along the medial portion 127 of the proximal body portion 120 may function to direct the load placed on the femoral component 110 on the medial, proximal portion 18 of the femur 10. The combination of the substantial curvature 125 of the proximal most one-third (1/3) of the femoral component 110 and the terminal flare portion 122 may function to load the proximal, medial portion 18 of the femur 10 when the femoral component 110 may be implanted in a femoral bone 10.

Referring now to FIGS. 9-13 and 26-27, the stem component 110 may include a protrusion or lateral fin 140 extending from the lateral side portion 128. The protrusion or lateral fin 140 may operate to contact the lateral cortex portion 19 of the femur 10 (illustrated best in FIGS. 1 and 2A). It will be appreciated that the lateral cortex portion 19 of the femur 10 is a hard, dense part of the femur 10 located laterally and the lateral cortex 19 is most pronounced in the proximal part of the femur 10 as illustrated in FIGS. 2 and 2A. The protrusion or lateral fin 140 may be configured to contact that hard cortical portion of the femur 10 to thereby resist torsional loads that may be placed on the stem component 110. Accordingly, the protrusion or lateral fin 140 may be contained entirely within the proximal most one-third (1/3) of the stem component 110, as illustrated in FIGS. 9, 11 and 26.

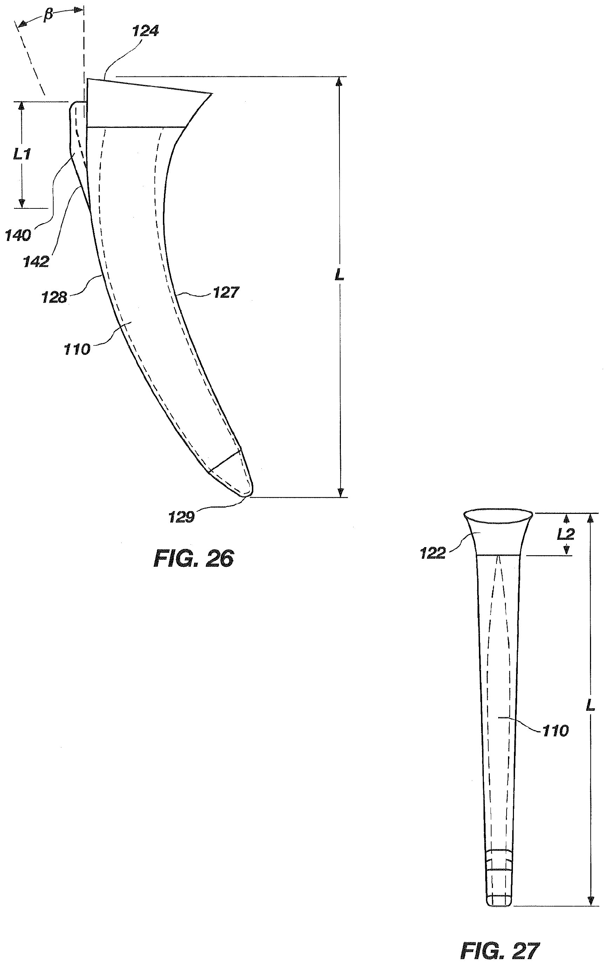

Referring now to FIG. 26, the protrusion or lateral fin 140 may include a length "L1" that may be within a range of about fifteen percent to about twenty-five percent of an overall length "L" of the stem component 110. The protrusion or lateral fin 140 may also include a tapered surface 142 that may taper in a proximal to distal direction. It will be appreciated that the taper may include an angle P that may be within a range of about ten degrees to about twenty-five degrees (or any angle within that range).

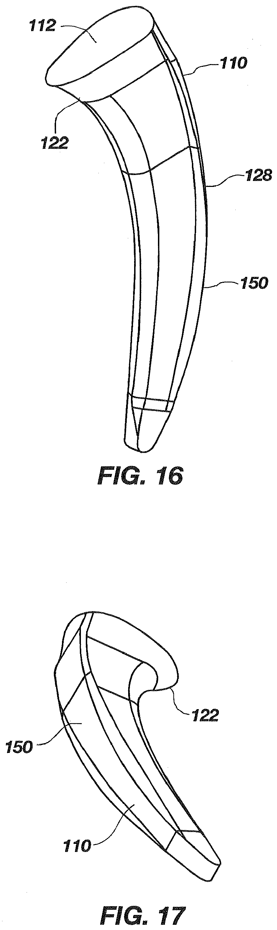

Referring now to FIGS. 14-19 and 22-23, in an alternative embodiment to the lateral fin 140, the stem component 110 may include on the lateral side portion 128 a substantially flat surface 150 that may curve, such that the lateral side portion 128 may be curved. The flat curved surface 150 of the lateral side portion 128 may be shaped as a wing back or T-back and may extend over a majority length "L" of the stem component 110. It is to be understood that the substantially flat surface 150 of the lateral side portion 128 may extend outwardly in an anterior and posterior direction and beyond the anterior side portion 114 and posterior side portion 116 (illustrated best in FIGS. 16,18 and 23). Further, the substantially flat surface 150 may extend along a majority length "L" of the stem component 110, thereby forming a flat, wing back for providing torsional stability to the stem component 110 when the stem component 110 is implanted within the femur 10.

As illustrated in FIGS. 22 and 23, the flat, wing back surface 150 may include a thickness "T1" that may be about five percent to about twenty-five percent of a thickness "T" of the stem component 110, which is the measurement between the anterior side 114 and the posterior side 116 of the stem component 110 (illustrated best in FIG. 23).

The stem component 110 may include a means for resisting torsional forces placed on the implant 50. It will be appreciated that the means for resisting torsional forces may be a number of features for resisting the natural torsional forces that are inherent in a hip joint. For example, the means for resisting torsional forces may be the protrusion or lateral fin 140 or it may be the wing back surface 150, both of which may be formed on the lateral side 128 of the stem component 110, or a flat surface 118 on the anterior and posterior sides 114 and 116.

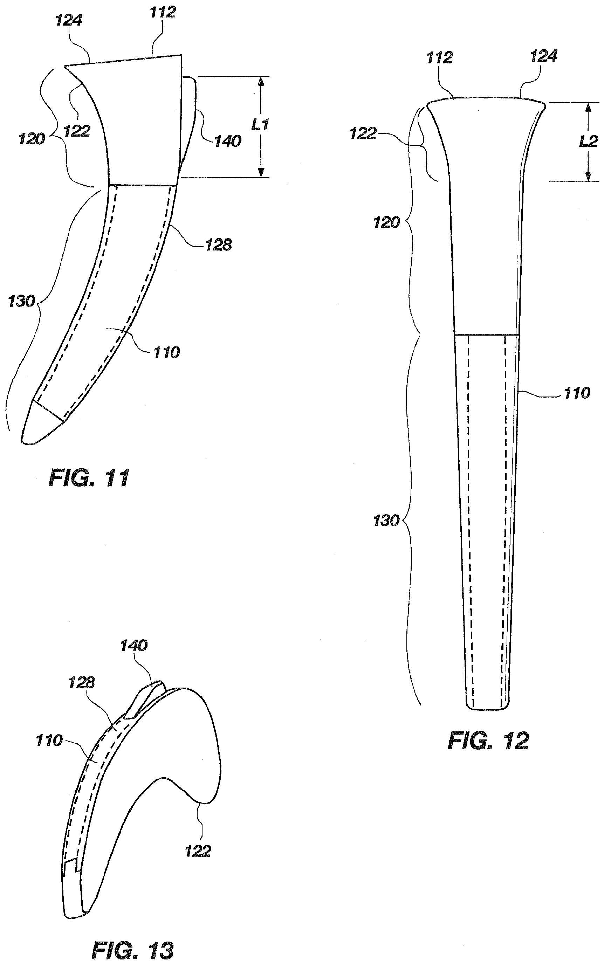

Referring now to FIGS. 12, 18, 21, 23, 25, and 27, the stem component 110 according to the invention further includes a means for internally contacting the medial calcar portion 18 of the femoral bone 10 in form of the terminal flare portion 122 that is configured and dimensioned to contact an internal medial calcar region 16 of the femoral bone 10. Because the largest amount of calcar bone in the femur is located medially, the terminal flare 122 flares out or extends radially outwardly in the medial, anterior and posterior dimensions near, at or from the proximal end 124 of the stem component 110 to contact the largest amount of calcar bone possible. The protrusion or the terminal flare portion 122 does not flare out on the lateral side of the stem component 110 to the same degree or in the same manner as it does in the medial, anterior and posterior sides. The result may be that a load placed on the implant 50, and specifically the femoral component 110, may be transferred medially from the femoral component 110 to the medial calcar region 16 of the femur 10.

The terminal flare portion 122 may extend near, at or from the proximal most end 124 of the stem component 110 for a length "L2" (illustrated best in FIG. 18). The length "L2" of the terminal flare portion 122 may be within a range of about two percent to about twenty percent of a length "L" of the entire stem component 110 as measured from the proximal end 124 to a distal end 129 and on the medial side 127 of the stem component 110. More specifically, the length "L2" may be within a range of about ten percent to about fifteen percent of the length "L" of the entire stem component 110. It is to be understood that all values within the specified ranges are to be considered within the scope of the present disclosure.

Referring now to FIGS. 24, 28, 30, and 31, it will be appreciated that any of the embodiments of the present disclosure may include a sagittal slot 160 that may be formed in the distal stem portion 130. Because the sagittal slot 160 may be formed in the distal portion 130 of the stem component 110, it may separate or split the stem component 110 into two pieces. The two pieces may be attached beneath a midline "M" of the stem component 110 as illustrated in FIGS. 24 and 28. In other words, the sagittal slot 160 may separate the medial side portion 127 from the lateral side portion 128 of the stem component 110 as illustrated in FIGS. 28, 30, and 31.

It will be appreciated that the sagittal slot 160 may allow the distal portion 130 of the stem component 110 to collapse to a small degree or extent to aid in fitting the distal portion 130 into the medullary canal of the femur 10 without damaging or protruding against the surrounding bone. In other words, the two separate pieces or sides of the distal portion 130 of the stem component 110 may be brought closer together as the distal portion 130 of the stem enters into the medullary canal and contacts other portions of the bone. Thus, the sagittal slot 160 may aid in implanting the stem component 110 into medullary canal of the femur 10 without damaging or protruding against the surrounding bone. The result may be a decrease in thigh pain for the patient.

It will be appreciated that the structure and apparatus disclosed herein is merely one example of a means for securing the modular neck component to the stem component, and it should be appreciated that any structure, apparatus or system for securing the modular neck component to the stem component that performs functions the same as, or equivalent to, those disclosed herein are intended to fall within the scope of a means for securing the modular neck component to the stem component, including those structures, apparatus or systems for securing the modular neck component to the stem component that are presently known, or which may become available in the future. Anything which functions the same as, or equivalently to, a means for securing the modular neck component to the stem component falls within the scope of this element.

It will be appreciated that the structure and apparatus disclosed herein is merely one example of a means for resisting torsional forces placed on the implant, and it should be appreciated that any structure, apparatus or system for resisting torsional forces placed on the implant that performs functions the same as, or equivalent to, those disclosed herein are intended to fall within the scope of a means for resisting torsional forces placed on the implant, including those structures, apparatus or systems for resisting torsional forces placed on the implant that are presently known, or which may become available in the future. Anything which functions the same as, or equivalently to, a means for resisting torsional forces placed on the implant falls within the scope of this element.

It will be appreciated that the structure and apparatus disclosed herein is merely one example of a means for internally contacting a medial calcar portion of a femoral bone, and it should be appreciated that any structure, apparatus or system for internally contacting a medial calcar portion of a femoral bone that performs functions the same as, or equivalent to, those disclosed herein are intended to fall within the scope of a means for internally contacting a medial calcar portion of a femoral bone, including those structures, apparatus or systems for internally contacting a medial calcar portion of a femoral bone that are presently known, or which may become available in the future. Anything which functions the same as, or equivalently to, a means for internally contacting a medial calcar portion of a femoral bone falls within the scope of this element.

In accordance with the features and combinations described above, a useful method of surgically locating a tissue sparing implant within a bone may comprise the steps of:
(a) providing the implant having a stem component and a terminal flare;
(b) surgically preparing a patient's proximal femur for receiving the implant, while preserving a majority portion of the patient's natural femoral neck, including a medial calcar portion;
(c) inserting the stem component of the implant into the surgically prepared proximal femur; and
(d) causing the terminal flare of the implant to internally contact the medial calcar portion of the femoral bone, such that load is transferred medially from the stem component to the medial calcar portion of the femur.

Those having ordinary skill in the relevant art will appreciate the advantages provided by the features of the present disclosure. For example, it is a potential feature of the present disclosure to provide a femoral component which is simple in design and manufacture and that places a load on the medial calcar portion of the femur. Another potential feature of the present disclosure is to provide such a femoral component having a terminal flare (i.e., a flare that is collarless). It another potential feature of the present disclosure to provide a femoral component having flat side portions on the anterior and posterior sides of the stem portion. It another potential feature of the present disclosure to provide a femoral component having short stem length and a substantial medial curvature on the proximal most one-third of the stem portion to place the load on the medial calcar region of the femur. It is yet another potential feature of the present disclosure to provide a femoral component having a lateral fin or a wing back or T-back. Finally, it is a potential feature of the present disclosure to provide a femoral component having a combination of the above features along with a sagittal slot.

In the foregoing Detailed Description of the Disclosure, various features of the present disclosure are grouped together in a single embodiment for the purpose of streamlining the disclosure. This method of disclosure is not to be interpreted as reflecting an intention that the claimed disclosure requires more features than are expressly recited in each claim. Rather, as the claims reflect, inventive aspects lie in less than all features of a single foregoing disclosed embodiment. Thus, the following claims are hereby incorporated into this Detailed Description of the Disclosure by this reference, with each claim standing on its own as a separate embodiment of the present disclosure.

It is to be understood that the above-described arrangements are only illustrative of the application of the principles of the present disclosure. Numerous modifications and alternative arrangements may be devised by those skilled in the art without departing from the scope of the present disclosure and the appended claims are intended to cover such modifications and arrangements.

## Claims

1. A tissue sparing implant comprising:
a stem (110) having a distal stem axis;
the stem (110) having a medial side (127), anterior side (114), posterior side (116), and a lateral side (128), the stem (110) further comprises a proximal end (124) having a terminal flare (122) flaring outwardly from said proximal end (124) on the medial (127), anterior (114) and posterior (116) sides of the stem (110), said terminal flare (122) having an exterior surface that forms an angle relative to the distal stem axis within a range of about seventy to about one-hundred and ten degrees, such that the terminal flare (122) contacts an internal medial calcar region (18) of a bone when implanted, such that load is transferred medially from the stem component (110) to the medial calcar region (18);
wherein the stem component (110) further comprises a medial curve (125) extending along a majority length of said stem component (110), wherein the medial curve (125) comprises at least three different radii of curvature that increase from a proximal end of said stem component to a distal end of said stem component and wherein the medial curve (125) is configured to model the natural medial curvature of a femoral neck of the natural femoral bone; and
**characterised in that** the terminal flare (122) does not flare outwardly on the lateral side (128) of the stem (110).

2. The tissue sparing implant of claim 1, wherein the stem component (110) comprises a length (L) from a top proximal most portion of the stem component (110) to a bottom distal most portion of the stem component (110) that is less than 150 millimeters.

3. The tissue sparing implant of claim 2, wherein the stem component (110) comprises a length (L) from a top proximal most portion of the stem component (110) to a bottom distal most portion of the stem component (110) that is within a range of about 100 millimeters to about 120 millimeters.

4. The tissue sparing implant of claim 1, wherein the stem component (110) comprises a flat surface (118) on an anterior side and a posterior side that extends substantially the entire length of the stem component (110).

5. The tissue sparing implant of claim 1, wherein the stem component (110) comprises a means for resisting torsional forces placed on the implant.

6. The tissue sparing implant of claim 5, wherein the means for resisting torsional forces is a wing back (150) formed on the lateral side of said stem component (110).

7. The tissue sparing implant of claim 6, wherein the wing back (150) is formed by a substantially flat lateral side portion extending beyond the anterior side and posterior side of the stem component (110).

8. The tissue sparing implant of claim 5, wherein the means for resisting torsional forces is a lateral fin (140) formed on the lateral side of said stem component (110).

9. The tissue sparing implant of claim 1, wherein the stem component (110) comprises a sagittal slot (160) formed in the distal most portion of said stem component (110) thereby separating a medial side from a lateral side of said stem component (110).

10. The tissue sparing implant of claim 8, wherein the lateral fin (140) is contained entirely within the proximal most one-third of the stem component (110).

11. The tissue sparing implant of claim 8, wherein the lateral fin (140) includes a length (L1) that is within a range of fifteen percent to twenty-five percent of the overall length (L) of the stem component (110).

12. The tissue sparing implant of claim 8, wherein the lateral fin (140) includes a tapered surface (142) that tapers in a proximal to distal direction.

13. The tissue sparing implant of claim 10, wherein the taper includes an angle ß that is within the range of ten degrees to twenty-five degrees.

14. The tissue sparing implant of claim 1, further comprising:
a neck component (100) comprising a straight longitudinal neck axis (A-A) extending through the neck component (100), wherein the stem component (110) is attachable to the neck component (100) and comprising a proximal end, a distal end, a proximal body portion (120), and a straight distal longitudinal stem axis (B-B) extending through the distal end of the stem component (110) and extending through a geometric center of a distal-most portion of said distal end; and
wherein the stem component further comprises a lateral side that is curved along a majority length of the stem, said lateral side also having a surface that is substantially flat along the majority length of the stem (110);
wherein the stem component (110) further comprises a length (L) that is less than 150 millimeters;
wherein an angle α is formed by an intersection of the straight neck axis when attached to said stem component (110) and the straight distal stem axis, wherein said angle α is within a range of about forty-five degrees and about sixty degrees, to thereby cause a proximal portion of the stem component (110) to have substantial medial curvature that is most pronounced in a proximal most one-third of the stem component, such that said proximal most portion of said stem component (110) has a pronounced deep curve;
wherein the proximal body portion (120) comprises a terminal flare (122) projecting radially outwardly from an upper most portion of said proximal body portion (120), wherein a length of the terminal flare (122) is within a range of about two percent to about twenty percent of the length of the stem component (110).

## Patentansprüche

1. Gewebeschonendes Implantat, umfassend:
einen Schaft (110) mit einer distalen Schaftachse;
den Schaft (110) mit einer medialen Seite (127), anterioren Seite (114), posterioren Seite (116) und einer lateralen Seite (128), wobei der Schaft (110) ferner ein proximales Ende (124) mit einer terminalen Aufweitung (122) umfasst, die sich nach außen von besagtem proximalem Ende (124) auf den medialen (127), anterioren (114) und posterioren (116) Seiten des Schafts (110) aufweitet, wobei besagte terminale Aufweitung (122) eine Außenfläche aufweist, die einen Winkel relativ zur distalen Schaftachse innerhalb eines Bereichs von etwa siebzig bis etwa einhundertzehn Grad bildet, sodass die terminale Aufweitung (122) eine interne mediale Calcarregion (18) eines Knochens berührt, wenn implantiert, sodass Last medial von der Schaftkomponente (110) auf die mediale Calcarregion (18) transferiert wird;
worin die Schaftkomponente (110) ferner eine mediale Kurve (125) umfasst, die sich entlang eines Großteils der Länge von besagter Schaftkomponente (110) erstreckt, worin die mediale Kurve (125) mindestens drei verschiedene Krümmungsradien umfasst, die von einem proximalen Ende von besagter Schaftkomponente zu einem distalen Ende von besagter Schaftkomponente zunehmen und worin die mediale Kurve (125) konfiguriert ist, um die natürliche mediale Krümmung eines Schenkelhalses des natürlichen Oberschenkelknochens zu modellieren; und
**dadurch gekennzeichnet, dass** die terminale Aufweitung (122) sich nicht nach außen auf der lateralen Seite (128) des Schafts (110) aufweitet.

2. Gewebeschonendes Implantat nach Anspruch 1, worin die Schaftkomponente (110) eine Länge (L) von einem oberen proximalsten Abschnitt der Schaftkomponente (110) zu einem unteren distalsten Abschnitt der Schaftkomponente (110), die weniger als 150 Millimeter ist, umfasst.

3. Gewebeschonendes Implantat nach Anspruch 2, worin die Schaftkomponente (110) eine Länge (L) von einem oberen proximalsten Abschnitt der Schaftkomponente (110) zu einem unteren distalsten Abschnitt der Schaftkomponente (110), die innerhalb eines Bereichs von etwa 100 Millimeter bis etwa 120 Millimeter liegt, umfasst.

4. Gewebeschonendes Implantat nach Anspruch 1, worin die Schaftkomponente (110) eine flache Oberfläche (118) auf einer anterioren Seite und einer posterioren Seite, die sich im Wesentlichen über die gesamte Länge der Schaftkomponente (110) erstreckt, umfasst.

5. Gewebeschonendes Implantat nach Anspruch 1, worin die Schaftkomponente (110) ein Mittel zum Widerstand gegen dem Implantat auferlegte Torsionskräfte umfasst.

6. Gewebeschonendes Implantat nach Anspruch 5, worin das Mittel zum Widerstand gegen Torsionskräfte ein auf der lateralen Seite der besagten Schaftkomponente (110) gebildeter Flügel (150) ist.

7. Gewebeschonendes Implantat nach Anspruch 6, worin der Flügel (150) durch einen im Wesentlichen flachen lateralen Seitenabschnitt gebildet wird, der sich jenseits der anterioren Seite und posterioren Seite der Schaftkomponente (110) erstreckt.

8. Gewebeschonendes Implantat nach Anspruch 5, worin das Mittel zum Widerstand gegen Torsionskräfte eine laterale Rippe (140) ist, die auf der lateralen Seite der besagten Schaftkomponente (110) gebildet wird.

9. Gewebeschonendes Implantat nach Anspruch 1, worin die Schaftkomponente (110) einen im distalsten Abschnitt der besagten Schaftkomponente (110) gebildeten sagittalen Schlitz (160) umfasst, wodurch eine mediale Seite von einer lateralen Seite der besagten Schaftkomponente (110) getrennt wird.

10. Gewebeschonendes Implantat nach Anspruch 8, worin die laterale Rippe (140) gänzlich innerhalb des proximalsten Drittels der Schaftkomponente (110) enthalten ist.

11. Gewebeschonendes Implantat nach Anspruch 8, worin die laterale Rippe (140) eine Länge (L1) beinhaltet, die innerhalb eines Bereichs von fünfzehn Prozent bis fünfundzwanzig Prozent der Gesamtlänge (L) der Schaftkomponente (110) liegt.

12. Gewebeschonendes Implantat nach Anspruch 8, worin die laterale Rippe (140) eine sich verjüngende Oberfläche (142) beinhaltet, die sich in proximaler zu distaler Richtung verjüngt.

13. Gewebeschonendes Implantat nach Anspruch 10, worin die Verjüngung einen Winkel β beinhaltet, der innerhalb des Bereichs von zehn Grad bis fünfundzwanzig Grad liegt.

14. Gewebeschonendes Implantat nach Anspruch 1, ferner umfassend:
eine Halskomponente (100) umfassend eine gerade longitudinale Halsachse (A-A), die durch die Halskomponente (100) verläuft, worin die Schaftkomponente (110) an der Halskomponente (100) anbringbar ist und ein proximales Ende, ein distales Ende, einen proximalen Körperabschnitt (120) und eine gerade distale longitudinale Schaftachse (B-B), die durch das distale Ende der Schaftkomponente (110) verläuft und durch eine geometrische Mitte eines distalsten Abschnitts von besagtem distalem Ende verläuft, umfasst; und
worin die Schaftkomponente ferner eine laterale Seite umfasst, die entlang eines Großteils der Länge des Schafts gekrümmt ist, wobei besagte laterale Seite auch eine Oberfläche aufweist, die im Wesentlichen flach entlang des Großteils der Länge des Schafts (110) ist;
worin die Schaftkomponente (110) ferner eine Länge (L) umfasst, die weniger als 150 Millimeter ist;
worin ein Winkel α durch eine Überschneidung der geraden Halsachse, wenn an besagter Schaftkomponente (110) angebracht, und der geraden distalen Schaftachse gebildet wird, worin besagter Winkel α innerhalb eines Bereichs von etwa fünfundvierzig Grad und etwa sechzig Grad liegt, um dadurch zu bewirken, dass ein proximaler Abschnitt der Schaftkomponente (110) erhebliche mediale Krümmung aufweist, die in einem proximalsten Drittel der Schaftkomponente am ausgeprägtesten ist, sodass besagter proximalster Abschnitt von besagter Schaftkomponente (110) eine ausgeprägte tiefe Kurve aufweist;
worin der proximale Körperabschnitt (120) eine terminale Aufweitung (122) umfasst, die radial nach außen aus einem obersten Abschnitt von besagtem proximalem Körperabschnitt (120) hervorsteht, worin eine Länge der terminalen Aufweitung (122) innerhalb eines Bereichs von etwa zwei Prozent bis etwa zwanzig Prozent der Länge der Schaftkomponente (110) liegt.

## Revendications

1. Implant préservant les tissus, comprenant :
une tige (110) ayant un axe de tige distale ;
la tige (110) ayant un côté médial (127), un côté antérieur (114), un côté postérieur (116) et un côté latéral (128), la tige (110) comprend en outre une extrémité proximale (124) ayant un évasement terminal (122) s'évasant vers l'extérieur à partir de laite extrémité proximale (124) sur les côtés médial (127), antérieur (114) et postérieur (116) de la tige (110), ledit évasement terminal (122) ayant une surface extérieure qui forme un angle, par rapport à l'axe de tige distale, dans une gamme allant d'environ soixante-dix à environ cent-dix degrés, de sorte que l'évasement terminal (122) entre en contact avec une région corticale médiale interne (18) d'un os lorsqu'il est implanté, de sorte que la charge soit transférée médialement de l'élément de tige (110) à la région corticale médiale (18) ;
dans lequel l'élément de tige (110) comprend en outre une courbure médiale (125) s'étendant dans le sens d'une longueur principale dudit élément de tige (110), dans lequel la courbure médiale (125) comprend au moins trois rayons de courbure différents qui augmentent d'une extrémité proximale dudit élément de tige à une extrémité distale dudit élément de tige et dans lequel la courbure médiale (125) est conçue pour modéliser la courbure médiale naturelle d'un col fémoral de l'os fémoral naturel ; et
**caractérisé en ce que** l'évasement terminal (122) ne s'évase pas vers l'extérieur sur le côté latéral (128) de la tige (110).

2. Implant préservant les tissus selon la revendication 1, dans lequel l'élément de tige (110) comprend une longueur (L), d'une partie supérieure la plus proximale de l'élément de tige (110) à une partie inférieure la plus distale de l'élément de tige (110), qui est de moins de 150 millimètres.

3. Implant préservant les tissus selon la revendication 2, dans lequel l'élément de tige (110) comprend une longueur (L), d'une partie supérieure la plus proximale de l'élément de tige (110) à une partie inférieure la plus distale de l'élément de tige (110), qui se situe dans une gamme allant d'environ 100 millimètres à environ 120 millimètres.

4. Implant préservant les tissus selon la revendication 1, dans lequel l'élément de tige (110) comprend une surface plate (118), sur un côté antérieur et un côté postérieur, qui s'étend essentiellement sur toute la longueur de l'élément de tige (110).

5. Implant préservant les tissus selon la revendication 1, dans lequel l'élément de tige (110) comprend un moyen pour résister aux forces de torsion qui s'exercent sur l'implant.

6. Implant préservant les tissus selon la revendication 5, dans lequel le moyen pour résister aux forces de torsion est un empennage (150) formé sur le côté latéral dudit élément de tige (110).

7. Implant préservant les tissus selon la revendication 6, dans lequel l'empennage (150) est formé par une partie du côté latéral essentiellement plate faisant saillie sur le côté antérieur et le côté postérieur de l'élément de tige (110).

8. Implant préservant les tissus selon la revendication 5, dans lequel le moyen pour résister aux forces de torsion est une ailette latérale (140) formée sur le côté latéral dudit élément de tige (110).

9. Implant préservant les tissus selon la revendication 1, dans lequel l'élément de tige (110) comprend une rainure sagittale (160) formée dans la partie la plus distale dudit élément de tige (110), séparant ainsi un côté médial d'un côté latéral dudit élément de tige (110).

10. Implant préservant les tissus selon la revendication 8, dans lequel l'ailette latérale (140) est entièrement contenue dans le tiers le plus proximal de l'élément de tige (110).

11. Implant préservant les tissus selon la revendication 8, dans lequel l'ailette latérale (140) présente une longueur (L1) qui se situe dans une gamme allant de quinze pour cent à vingt-cinq pour cent de la longueur totale (L) de l'élément de tige (110).

12. Implant préservant les tissus selon la revendication 8, dans lequel l'ailette latérale (140) présente une surface effilée (142) qui s'effile dans une direction proximale à distale.

13. Implant préservant les tissus selon la revendication 10, dans lequel l'effilement présente un angle β qui se situe dans la gamme allant de dix degrés à vingt-cinq degrés.

14. Implant préservant les tissus selon la revendication 1, comprenant en outre :
un élément de col (100) comprenant un axe longitudinal de col droit (A-A) s'étendant à travers l'élément de col (100), dans lequel l'élément de tige (110) peut être attaché à l'élément de col (100) et comprenant une extrémité proximale, une extrémité distale, une partie de corps proximale (120), et un axe longitudinal de tige distale droite (B-B) s'étendant à travers l'extrémité distale de l'élément de tige (110) et s'étendant à travers un centre géométrique d'une partie la plus distale de ladite extrémité distale ; et
dans lequel l'élément de tige comprend en outre un côté latéral qui est courbé dans le sens d'une longueur principale de la tige, ledit côté latéral ayant également une surface qui est essentiellement plate dans le sens de la longueur principale de la tige (110) ;
dans lequel l'élément de tige (110) comprend en outre une longueur (L) qui est de moins de 150 millimètres ;
dans lequel un angle α est formé par une intersection entre l'axe de col droit lorsqu'il est fixé audit élément de tige (110) et l'axe de tige distale droite, dans lequel ledit angle α se situe dans une gamme allant d'environ quarante-cinq degrés à environ soixante degrés, pour faire en sorte qu'une partie proximale de l'élément de tige (110) ait une courbure médiale significative qui est plus prononcée dans un tiers le plus proximal de l'élément de tige, de sorte que ladite partie la plus proximale dudit élément de tige (110) ait une courbure intense prononcée ;
dans lequel la partie de corps proximale (120) comprend un évasement terminal (122) faisant saillie radialement vers l'extérieur à partir d'une partie la plus haute de ladite partie de corps proximale (120), dans lequel une longueur de l'évasement terminal (122) se situe dans une gamme allant d'environ deux pour cent à environ vingt pour cent de la longueur de l'élément de tige (110).
